# EUROPEAN PATENT SPECIFICATION

(11) **EP 1 444 623 B1**
(45) Date of publication and mention of the grant of the patent: **19.04.2006**
(21) Application number: 02768638.5
(22) Date of filing: 20.08.2002
(51) Int. Cl.: G06Q 10/00, G06F 19/00

(54) **PRESCRIPTION FULFILLMENT SYSTEM AND METHOD**
REZEPTAUSFÜHRUNGSSYSTEM UND VERFAHREN
PROCEDE ET SYSTEME DE REALISATION D'UNE PRESCRIPTION

(30) Priority: 05.11.2001 US 8488
(43) Date of publication of application: 11.08.2004
(73) Proprietor: Holz, Siegfried K., Lakeland, FL 33913 (US)
(72) Inventor: Holz, Siegfried K., Lakeland, FL 33913 (US)
(74) Representative: Legg, Cyrus James Grahame
(86) International application number: PCT/US2002/026574
(87) International publication number: WO 2003/040980

(56) References cited:
- WO-A-97/04712
- US-A- 5 077 666
- US-A- 5 291 399
- US-A- 5 301 105
- US-A- 5 659 741
- US-A- 5 924 074
- US-A1- 2001 001 144
- US-A1- 2001 034 613
- US-A1- 2002 026 332
- US-A1- 2002 099 595
- 'Consumers embrace online health' PERSONALMD PRESS RELEASE, [Online] 26 January 2000, XP002960934 Retrieved from the Internet: <URL:www.personalmd.com/press29_article.sht ml>
- 'PersonalMD.com unveils three new personalized services' PERSONALMD PRESS RELEASE, [Online] 11 February 2000, XP002957872 Retrieved from the Internet: <URL:http://www.personalmd.com/press33_arti cle.shtml>
- 'Techneos announces sophisticated survey software for handheld computers' TECHNEOS SYSTEMS: NEWS, [Online] 05 May 2000, XP002953664 Retrieved from the Internet: <URL:www.techneos.com/news_052500.html>

## Description

This invention relates generally to the field of medicine and more particularly to systems and methods for providing portable patient medical history and prescription fulfillments in conjunction with medical examinations by physicians.

Although the record keeping functions associated with routine or emergency office visits with a physician have greatly improved, nonetheless the inconvenience of the lack of centrally located portable medical histories for patients and the easy and convenient prescription fulfillment for prescriptions ordered by a physician at the time of physical examination of a patient are still somewhat archaic and cumbersome.

U.S. patent 4,695,954, invented by Rose et al., teaches an apparatus and method for dispensing medications utilizing a portable memory device used to store data representing prescription information. A container having a plurality of individual compartments is filled with medication at the pharmacy in accordance with the prescription information provided on the portable memory device.

An automatic system for printing prescription forms is disclosed in U.S. patent 5,528,021 invented by Lassus et al. This invention uses a chip (personal memory) card (PMC) reader with a microprocessor and a printer and is aimed at simplifying the operations of transcribing the information on the prescription and limiting the risks of prescription fraud.

Thornton, in U.S. patent 5,628,530, discloses a method and system for tracking prescribed starter drug samples dispensed to patients. The system employs the use of a smart card which has been encoded with the prescription information and is decoded at the pharmacy via a conventional card reader and input into the pharmacy computer for providing tracking information for the dispensed starter drug samples.

U.S. Patent 5,737,539 to Edelson et al. teaches a prescription creation system permitting creation of a single prescription to be automatically divided for fulfillment of one portion quickly and another portion by remote mail order. This system further has the ability to access remote source databases for presentation to the prescriber of relevant, authorized and drug formulary and patient history.

A prescription management system is disclosed in U.S. patent 5,845,255 invented by Mayaud. This invention teaches use of a personal digital assistant (PDA) for use by a physician to input prescribed treatment and patient records with privacy controls and online access to comprehensive drug information. The preferred embodiment relates to a computer-implemented prescription system to assist physicians in prescribing and reviewing drugs.

Sattizahn et al discloses in U.S. Patent 5,884,273 a hand-held microcomputer with an attached printer that receives input from a physician and prints out a legible prescription slip. The prescription slip contains relevant information for the patient and pharmacists.

U.S. Patent 5,991,731 relates to clinical studies and teaches a method and system for interactive prescription and distribution of prescriptions in conducting those studies. The system communicates data over the Internet to determine patient eligibility, randomization and initial prescriptions which can be adjusted by the physician online. The prescription is then printed out for signature and send electronically to a distribution center.

An electronic pocket pillbox and prescription writing apparatus is taught in U.S. patent 6,032,085 issued to Laurent et al. A physician prepares a prescription utilizing a computer which loads the prescription file into the memory of a smart card which is put into the pillbox to supply signals to the pillbox advising the user to take the medication.

U.S. patent 6,067,524 discloses a system for automatically generating advisory information for distribution to pharmacy patients. The system generates advisory messages to patents including patient-specific information to a data record which is transmitted between a third party computer and a pharmacy computer during a pharmacy transaction.

A computer-based pharmaceutical practice management system and healthcare management system for use by a pharmacy is disclosed in U.S. patent 6,112,182 issued to Akers et al. Health conditions of a patent, drugs taken by the patient and other information gathered by the pharmacist will automatically initiate related actions handled by processes of the healthcare management system.

Barry et al., in U.S. patent 6,188,988 discloses a system, method and computer program for treatment for known diseases. The method comprises inputting patient information onto a computer, generating in the computer a listing of treatment regimens for the patient and generating advisory information for one or more treatment regimens.

An electronic clinical recording system is shown in U.S. patent 6,272,470 issued To Teshima. The system comprises a portable storage medium for storing the patient's record and an element for reading the patient's record, the major object being to provide an electronic clinical recording system for a hospital information system enabling the sharing of medical information at low cost without concern of type of equipment.

In U.S. patent 6,281,798, Laurent et al. teaches a multi-compartment electronic pocket pillbox with a microprocessor having memory loaded with prescription data. This pillbox further includes a control circuit for display, circuitry for loading into the memory data contained in a detachable data medium and detectors for ascertaining from which compartment the pill was withdrawn.

U.S. patent 5,659,741 and international publication WO 97/04712 disclose the use of a personal memory card carried by a patient onto which various data about the patient and his treatment can be written and read from.

This invention is directed to a system and method for preparing and fulfilling a medication prescription written by a physician at the time of conducting a physical examination of a patient. The system includes a personal memory card (PMC) for the benefit of, and carried by, the patient which is configured to include a modifiable stored memory including the patient's personal information, consulting physician information, accessible pharmacy information, medical history, insurer information and authentification code. A PMC reader/writer receives the PMC and viewably presents the stored memory on a personal digital assistant (PDA) in conjunction with the examination of the patient by the physician. After the examination, stored memory is modifiable by the physician via the PDA to include any new medication prescriptions required and additional medical history to form an updated stored memory. Another PMC reader/writer connected to an office computer of the physician is configured to receive and electronically transfer the updated stored memory to a remotely located central host server. The host server is configured to receive and store the updated stored memory and to electronically transmit a prescription fulfillment request for any new prescriptions contained within the updated stored memory to a selected one of the accessible pharmacies identified in the stored memory and to electronically transmit a confirmation back to the office computer advising that any new prescriptions are fulfilled. Appropriate insurer information may also be electronically transferred from the host server to a designated insurer.

It is therefore an object of this invention to provide a system and method for providing a portable modifiable memory card for patients which is utilized by a physician during an office visit to amplify or modify prescriptions for medicine, to effect prescription fulfillment and to establish and update stored medical history of each patient both on the memory card and in the computer system of a host server.

It is another object of this invention to provide a system and method for physical examination, medical history update and prescription fulfillment all of which are accomplished while the patient is at the office of the physician.

In accordance with these and other objects which will become apparent hereinafter, the instant invention will now be described with reference to the accompanying drawings.
Figure 1 is a schematic view of one aspect of the invention associated with the physical examination by a physician.
Figure 2 is a schematic view of another portion of the invention including the physician's office computer, the central host server, third party pharmacies and insurer's computers.
Figure 3 is a block flow diagram of the invention with respect to the patient physical examination by a physician, the authorization of new prescriptions and updating medical history.
Figure 4 is a flow diagram of the invention related to the transferring of updated stored medical history and new prescriptions from the office of the examining physician to a central host server and thereafter to a selected pharmacy for prescription fulfillment.

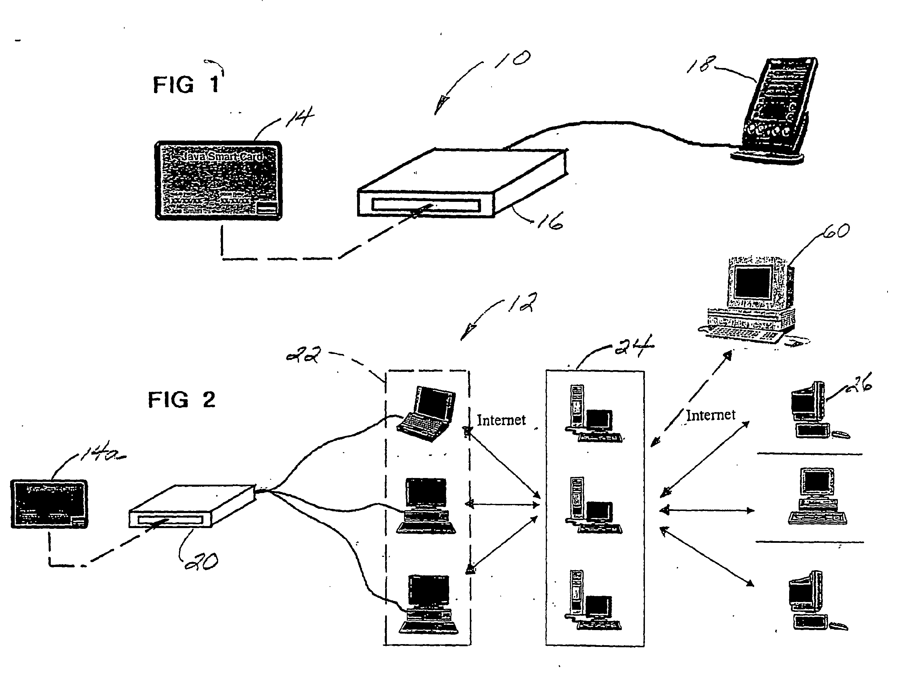

Referring now to the drawings and particularly to Figure 1, a portion of the system is there shown generally at numeral 10 and includes a personal memory card (PMC) 14 known as a java card or a SMART CARD having a programmable memory chip embedded therein. The PMC 14 is initially issued to a patient and is programmed to include stored memory related to the name, address and other personal information of the patient, information regarding physicians that have attended to the patient, including the name of each physician, the area of expertise, location, phone number, etc. This stored memory also includes information regarding pharmacies to which the patient has access including the name, location, phone number, etc. of each designated pharmacy. The patient's medical history, past medical problems, known allergies and reactions and current medication are further included. Finally, an authentification code is added to the stored memory which serves to insure that the person bearing and presenting the PMC is indeed the proper patient identified in the stored memory of that PMC.

Still referring to Figure 1, when the patient undergoes a physical examination by a physician, the PMC **14** is presented at the time of the commencement of the examination and the physician then inserts the PMC **14** into a PMC reader/writer **16** which reads and conveys the stored memory to a personal digital assistant PDA) **18** such as a PALM PILOT and the like. The physician may then review the entire medical history of the patient before beginning the physical examination. At the end of the examination, the physician will typically then, using the PDA **18,** modify the stored memory by entering his diagnosis of new medical conditions and by writing one or more new prescription authorizations into the updated stored memory. When this updating is completed by the physician, the PMC **14** is removed from the PMC reader/writer **16** and returned to the patient.

Referring now to Figure 2, the patient then moves to the central office area of the physician's facility and presents the updated PMC **14a** to personnel in the office. The updated PMC **14a** is inserted into another PMC reader/writer **20,** the updated stored memory then transferred to one of the office computers **22.** Thereafter, while the patient waits, the updated stored memory is transmitted via Internet connection to a central host server **24.** This host server **24** includes a bank of appropriate computer equipment to receive and store the updated stored memory and to discern that new prescriptions and other information have been entered into the updated stored memory. The host server **24** then seeks out by Internet connection one of the pharmacies designated in the updated stored memory with which to place a prescription fulfillment request. This is done by the Internet to locate the first pharmacy **26** from the stored memory listing of pharmacies convenient to the patient which is recognized as being capable of prescription fulfillment by return confirmation to the host server **24.**

When the host server **24** receives prescription fulfillment request confirmation, a fulfillment confirmation is transmitted by the Internet back to the office computer **22.** When that confirmation is received, the patient's PMC is returned to him or her to complete the office visit.

As an additional function of the host server **24,** electronic communication by the Internet may also be facilitated to transmit billing information concerning the physical examination and new prescription requests to an insurer computer 60. This information transfer to the insurance computer **60** may be utilized to expedite the billing and payment of the charges for the physical examination and to advise the insurance computer facility **60** that the new prescription has been authorized. Ideally, by including information transfer between the host server **24** and the insurance computer **60,** expedited payment authorization for these medical services and prescriptions will be substantially expedited and improved upon from an efficiency standpoint.

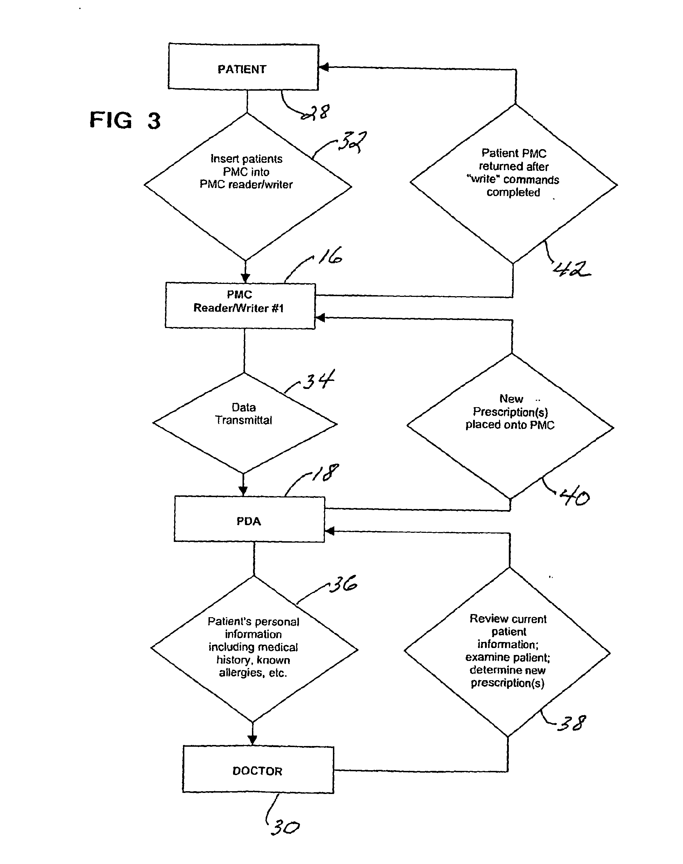

A flow diagram with respect to the physical examination proceeding between the physician **30** and the patient **28** is depicted in block form in Figure 3. The patient provides his PMC to the physician at **32** who then inserts the PMC into the PMC reader **16.** Data is transmitted at **34** to the doctor's PDA **18** whereupon he reviews the current stored memory information at **36** and then commences the physical examination of the patient **28.** After the physical examination is completed and any new prescriptions are determined to be necessary at **38,** the physician **30** then enters these new prescriptions and updated medical history into the PDA **18.** These updates are then transferred at **40** into the permanent stored memory of the PMC by the PMC reader/writer **16.** After the stored memory has been updated to establish an updated stored memory at **42,** the PMC is returned to the patient.

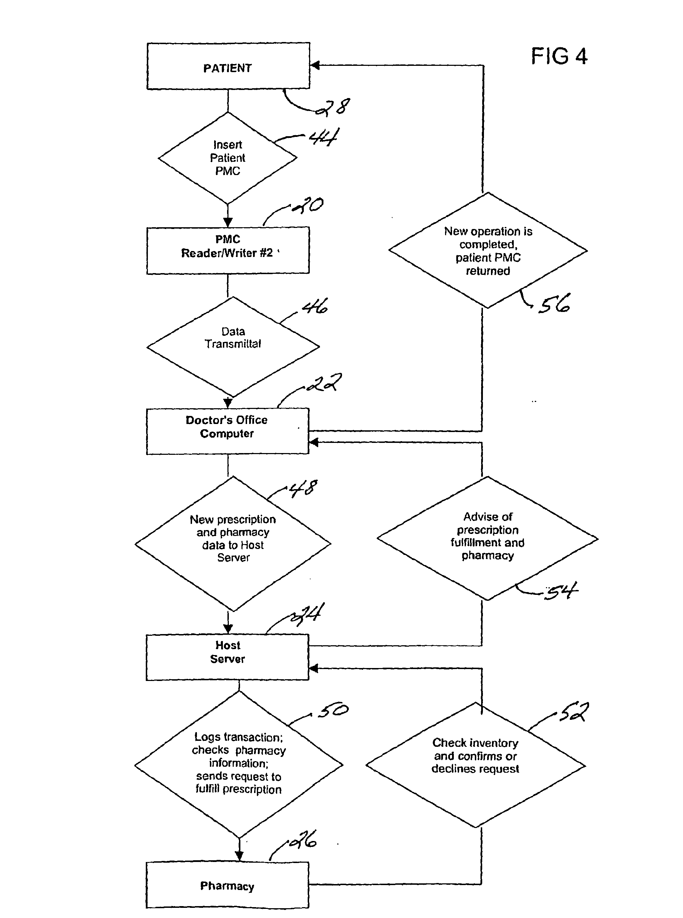

Referring lastly to Figure 4, once the patient **28** has completed the physical examination and updated stored memory has been entered onto his or her PMC, the patent then moves into the main office area where the physician is located and personnel will again accept his PMC and insert it at **44** into a second PMC reader/writer **20.** The updated stored memory will be transmitted at **46** into the office computer **22.** Internet connection is then established with the host server **24** and the new prescription and pharmacy data and updated medical history will be transmitted electronically at **48** into the host server **24.**

The host server **24** will then establish Internet connection with a preselected pharmacy **26** from the updated stored memory on the PMC at **50.** The pharmacy **26** will then check and confirm prescription inventory at **52** and will either confirm the fulfillment request or decline the request due to unavailability of the medication ordered. Once a pharmacy **26** has confirmed fulfillment, the host server **24** will advise of prescription fulfillment and the details and location of the pharmacy at **54** to the doctor's office computer **22.** As soon as the doctor's office computer **22** receives prescription fulfillment confirmation, the PMC will be returned to the patient **28** at **56** to complete the patient's office visit.

While the instant invention has been shown and described herein in what are conceived to be the most practical and preferred embodiments, it is recognized that departures may be made therefrom within the scope of the invention, which is defined by the claims.

## Claims

1. A system for preparing and fulfilling a patient's prescription written by a physician for medication comprising:
a personal memory card PMO (14) for the benefit of, and carried by the patient, said PMC configured to include a modifiable stored memory including the patient's personal information, consulting physician information, accessible pharmacy information, medical history and authentification code;
a first PMC eader/write (16) for receiving and viewably presenting said stored memory on a personal digital assistant PDA (18) in conjunction with a physical examination of the patient by the physician, said stored memory being modifiable by the physician via said PDA to include any new medication prescriptions and additional medical history to form an updated stored memory;
a second PMC reader/writer (20) connected to an office computer (22) of the physician configured to receive and electronically transfer said updated stored memory to a remotely located central host server (24);
said host server configured to store said updated stored memory and to electronically transmit a prescription fulfillment request for any new prescriptions contained within said updated stored memory to a selected one of the accessible pharmacies (26) and to electronically transmit a confirmation back to said office computer advising that the new prescriptions are fulfilled.

2. A method of preparing and fulfilling a medication prescription written by a physician for a patient at the time of physical examination comprising the steps of:
A. providing a personal memory card PMC (14) established for the benefit of, and carried by the patient, said PMC configured to include a stored memory including the patient's personal information, consulting physician information, accessible pharmacy information, medical history, insurer information and an authentification code;
B. reading said stored memory into a first PMC reader/writer (16) from said PMC and viewably presenting said stored memory on a personal digital assistant PDA (18) in conjunction with a physical examination of the patient by the physician;
C. modifying said stored memory of said PMC by the physician via entry on said PDA to include new medication prescriptions and additional medical history to form an updated stored memory;
D. reading said updated stored memory of said PMC via a second PMC reader/writer (20) into to an office computer (22) of the physician, said office computer configured to electronically transfer said updated stored memory to a remotely located central host server (24);
E. transferring said updated stored memory to the host server by the Internet;
F. transmitting a prescription fulfillment request electronically by said host server for new prescriptions contained within said updated stored memory to one of the accessible pharmacies (26), said host server configured to store said updated stored memory and for electronically transmitting said request;
G. transmitting a confirmation electronically from one of the pharmacies selected in step F back to said office computer advising that any new prescriptions are fulfilled.

3. The system of claim 1 wherein:
said stored memory of said PMC (14) is also configured to include insurer information;
said host server (24) is also configured to electronically transfer insurance information of said updated stored memory to an insurer (60) designated in said updated stored memory.

4. The method of claim 2, further comprising the steps of:
H. electronically transmitting appropriate insurer information by said host server (24) corresponding to the examination and new prescriptions.

## Revendications

1. Système pour préparer et exécuter une prescription d'un patient, rédigée par un médecin, pour des médicaments, comprenant :
une carte mémoire personnelle PMC (14) à l'intention du patient, et portée par lui, ladite PMC étant configurée pour inclure une mémoire enregistrée modifiable comprenant des informations personnelles du patient, des informations du médecin traitant, des informations de pharmacies accessibles, les antécédents médicaux et un code d'authentification ;
un premier dispositif de lecture / écriture (16) de PMC pour recevoir et présenter de manière visible ladite mémoire enregistrée sur un assistant numérique personnel PDA (18) en association avec un examen physique du patient par le médecin, ladite mémoire enregistrée pouvant être modifiée par le médecin par l'intermédiaire dudit PDA pour inclure toutes nouvelles prescriptions de médicaments et des antécédents médicaux supplémentaires pour former une mémoire enregistrée mise à jour ;
un second dispositif de lecture / écriture (20) de PMC connecté à un ordinateur de bureau (22) du médecin, configuré pour recevoir et transférer électroniquement ladite mémoire enregistrée mise à jour vers un serveur hôte central distant (24) ;
ledit serveur hôte étant configuré pour enregistrer ladite mémoire enregistrée mise à jour et pour transmettre électroniquement une demande d'exécution de prescription de toutes nouvelles prescriptions contenues à l'intérieur de ladite mémoire enregistrée mise à jour, à une sélectionnée des pharmacies accessibles (26) et pour transmettre électroniquement en retour une confirmation audit ordinateur de bureau en l'informant que les nouvelles prescriptions sont exécutées.

2. Procédé pour préparer et exécuter une prescription de médicaments écrite par un médecin à l'intention d'un patient au moment de l'examen physique, comprenant les étapes consistant à :
A. fournir une carte mémoire personnelle PMC (14) établie à l'intention du patient, et portée par lui, ladite PMC étant configurée pour inclure une mémoire enregistrée comprenant des informations personnelles du patient, des informations du médecin traitant, des informations de pharmacies accessibles, les antécédents médicaux, des informations d'assurance et un code d'authentification ;
B. lire ladite mémoire enregistrée dans un premier dispositif de lecture / écriture (16) de PMC à partir de ladite PMC et présenter de manière visible la mémoire enregistrée sur un assistant numérique personnel PDA (18) en association avec un examen physique du patient par le médecin ;
C. modifier ladite mémoire enregistrée de ladite PMC par le médecin par l'intermédiaire d'une entrée sur ledit PDA pour inclure de nouvelles prescriptions de médicaments et des antécédents médicaux supplémentaires pour former une mémoire enregistrée mise à jour ;
D. lire ladite mémoire enregistrée mise à jour de ladite PMC par l'intermédiaire d'un second dispositif de lecture / écriture (20) de PMC dans un ordinateur de bureau (22) du médecin, configuré pour transférer électroniquement ladite mémoire enregistrée mise à jour vers un serveur hôte central situé à distance (24) ;
E. transférer ladite mémoire enregistrée mise à jour au serveur hôte par Internet ;
F. transmettre électroniquement une demande d'exécution de prescription par ledit serveur hôte des nouvelles prescriptions contenues à l'intérieur de ladite mémoire enregistrée mise à jour, à l'une des pharmacies accessibles (26), ledit serveur hôte étant configuré pour enregistrer ladite mémoire enregistrée mise à jour et pour transmettre électroniquement ladite demande ;
G. transmettre en retour électroniquement une confirmation à partir de l'une des pharmacies sélectionnées dans l'étape F, audit ordinateur de bureau en l'informant que toutes les nouvelles prescriptions sont exécutées.

3. Système selon la revendication 1, dans lequel :
ladite mémoire enregistrée de ladite PMC (14) est également configurée pour inclure des informations d'assurance ;
ledit serveur hôte (24) est également configuré pour transférer électroniquement les informations d'assurance de ladite mémoire enregistrée mise à jour à un assureur (60) indiqué dans ladite mémoire enregistrée mise à jour.

4. Procédé selon la revendication 2, comprenant de plus les étapes consistant à :
H. transmettre électroniquement les informations d'assurance appropriées par ledit serveur hôte (24) correspondant à l'examen et aux nouvelles prescriptions.

## Patentansprüche

1. System für die Ausstellung und Ausführung eines Rezepts für einen Patienten für ein Arzneimittel, ausgestellt durch einen Arzt, umfassend:
eine persönliche Chipkarte PMC (personal memory card) (14) zum Nutzen des Patienten und von diesem bei sich getragen, wobei die PMC so gestaltet ist, dass sie modifizierbare gespeicherte Daten einschließt, welche die persönlichen Angaben zum Patienten, Informationen des konsultierten Arztes, Informationen über zugängliche Apotheken, die Krankengeschichte und einen Authentifizierungscode enthalten;
ein erstes PMC-Lese-/Schreibgerät (16) für die Aufnahme und sichtbare Darstellung der gespeicherten Daten auf einem Personal Digital Assistant PDA (18) in Verbindung mit einer allgemeinen körperlichen Untersuchung des Patienten durch den Arzt, wobei die gespeicherten Daten durch den Arzt mit Hilfe des PDA dergestalt modifizierbar sind, dass sie jedes neue Arzneimittel-Rezept und zusätzliche Angaben zur Krankengeschichte einschließen, um einen aktualisierten gespeicherten Datenbestand auszubilden;
ein zweites PMC-Lese-/Schreibgerät (20), welches an einen Bürocomputer (22) des Arztes angeschlossen ist, der so konfiguriert ist, dass er den aktualisierten gespeicherten Datenbestand aufnimmt und elektronisch zu einem entfernt befindlichen zentralen Host-Server (24) überträgt;
wobei der Host-Server so konfiguriert ist, dass er die aktualisierten gespeicherten Daten speichert und elektronisch ein Rezeptausführungsersuchen für neue Rezepte, die in den aktualisierten gespeicherten Daten enthalten sind, an eine ausgewählte Apotheke unter den Apotheken (26), auf die zugegriffen werden kann, übermittelt und elektronisch eine Bestätigung zurück zum Bürocomputer übermittelt und mitteilt, dass die neuen Rezepte ausgeführt werden.

2. Verfahren für die Ausstellung und Ausführung eines Rezepts für ein Arzneimittel, ausgestellt durch einen Arzt für einen Patienten zum Zeitpunkt der allgemeinen körperlichen Untersuchung, folgende Schritte umfassend:
A. Vorlage einer persönlichen Chipkarte PMC (14), ausgestellt zum Nutzen des Patienten und von diesem bei sich getragen, wobei die PMC so gestaltet ist, dass sie gespeicherte Daten einschließt, welche die persönlichen Angaben zum Patienten, Informationen des konsultierten Arztes, Informationen über zugängliche Apotheken, die Krankengeschichte, Angaben zum Versicherer und einen Authentifizierungscode enthalten;
B. Einlesen der gespeicherten Daten in ein erstes PMC-Lese-/Schreibgerät (16) von dieser PMC und sichtbare Darstellung der gespeicherten Daten auf einem Personal Digital Assistant PDA (18) in Verbindung mit einer allgemeinen körperlichen Untersuchung des Patienten durch den Arzt;
C. Modifizierung der gespeicherten Daten dieser PMC durch den Arzt durch die Eingabe in den PDA dergestalt, dass dieselben neue Arzneimittel-Rezepte und zusätzliche Angaben zur Krankengeschichte einschließen, um einen aktualisierten gespeicherten Datenbestand auszubilden;
D. Einlesen des aktualisierten gespeicherten Datenbestands der PMC mit Hilfe eines zweiten PMC-Lese-/Schreibgeräts (20) in einen Bürocomputer (22) des Arztes, wobei der Bürocomputer so konfiguriert ist, dass er den aktualisierten gespeicherten Datenbestand elektronisch zu einem entfernt befindlichen zentralen Host-Server (24) überträgt;
E. Übertragung des aktualisierten gespeicherten Datenbestands an den Host-Server durch das Internet;
F. elektronische Übermittlung eines Rezeptausführungsersuchens durch den Host-Server für neue Rezepte, die in den aktualisierten gespeicherten Daten enthalten sind, an eine der Apotheken (26), auf die zugegriffen werden kann, wobei der Host-Server so konfiguriert ist, dass er den aktualisierten gespeicherten Datenbestand speichert und das Ersuchen elektronisch übermittelt;
G. elektronische Übermittlung einer Bestätigung durch eine der in Schritt F ausgewählten Apotheken zurück an den Bürocomputer mit der Mitteilung, dass neue Rezepte ausgeführt werden.

3. System von Anspruch 1, **dadurch gekennzeichnet, dass**:
der gespeicherte Datenbestand der PMC (14) ebenfalls so konfiguriert ist, dass er Informationen zum Versicherer einschließt;
der Host-Server (24) ebenfalls so konfiguriert ist, dass er elektronisch Versicherungsinformationen des aktualisierten gespeicherten Datenbestands an einen Versicherer (60) überträgt, welcher im aktualisierten gespeicherten Datenbestand angegeben ist.

4. Verfahren nach Anspruch 2, welches weiterhin den folgenden Schritt umfasst:
H. elektronische Übermittlung entsprechender Versicherer-Informationen durch den Host-Server (24), welche sich in Übereinstimmung mit der Untersuchung und mit neuen Rezepten befinden.
